# EUROPEAN PATENT SPECIFICATION

(11) **EP 1 751 173 B1**
(45) Date of publication and mention of the grant of the patent: **05.11.2008**
(21) Application number: 04735464.2
(22) Date of filing: 31.05.2004
(51) Int. Cl.: C07J 9/00

(54) **PROCESS FOR THE RECOVERY OF STEROLS FROM ORGANIC MATERIAL**
VERFAHREN ZUR RÜCKGEWINNUNG VON STEROLEN AUS ORGANISCHEM MATERIAL
PROCEDE POUR LA RECUPERATION DE STEROLS SUR MATERIAU ORGANIQUE

(43) Date of publication of application: 14.02.2007
(73) Proprietor: Cognis IP Management GmbH, 40589 Düsseldorf (DE)
(72) Inventor: SATO, Setsuo, Jardim Santa Maria, Jacareí, SP (BR); ALBIEZ, Wolfgang, D-41468 Neuss (DE); ARAUJO, Alexsander, S., Parque Industrial, 12237-000 Sao José dos Campos (BR); BUENO DE ALMEIDA, Wanderson, 12216-590 Sao Jose dos Campos - SP (BR)
(86) International application number: PCT/BR2004/000082
(87) International publication number: WO 2005/116048

(56) References cited:
- EP-A- 0 260 243
- WO-A-94/03473
- DE-A1- 10 038 457
- US-A- 4 374 776

## Description

The present invention generally belongs to the area of chemical processes of isolating and purifying constituents from natural sources and, in particular, it relates to a new method for separating concentrating and purifying sterols from organic material.

### State of the art

Numerous methods have been described for the recovery of sterols from organic material converting sterol fatty esters into free sterols with subsequent purification by distillation and crystallization.
The separation of sterols by transesterification and saponification steps followed by further esterification or solvent extraction often requires organic solvents, results to large amounts of salts as waste and usually needs many process steps, so that the process is less economic and less environmentally friendly and results in relatively low yields.

The US-patent US 6,344,573 B1 relates to a process for the extraction and concentration of unsaponifiables substances from residues of animal or vegetable products. This process does not require organic solvents but involves several process steps creating a high amount of byproducts.
The European patent application EP 1291355 A1 discloses a process for recovering sterols and/or wax alcohols from a crude tall oil material containing sterols and/or wax alcohols in esterified form and fatty and/or rosin acids and optionally sterols and/or wax alcohols in free form. Said method is comprising the steps of: converting free acids in the source material to corresponding salts, removing water if present, transesterifying the esterified sterols and/or wax alcohols present in the dry material obtained in step a or step b to liberate sterols and/or wax alcohols, evaporative fractionating the transesterified material, and isolating sterols and/or wax alcohols from the obtained fractions or the residue.

After liberating the bonded sterols, the material is submitted to a fractionation step to separate the sterols from the other components. Sterols remain in the residue stream and light end components are distilled off. This process consists of a large number of different steps bearing the risk to decrease the final yield of free sterols.
A high efficiency continuous process for recovering high purity sterol mixtures from organic material including tall oil pitch is described in the European patent application EP 1081156 A2 comprising the steps of distillation, crystallization and recirculation of the mother liquor residue. In order to achieve the high purity many production steps and the use of organic solvents was necessary.
The International application WO 00/64921 discloses a process to purify sterols from natural sources by complexation with a metal salt, this process has less process steps, but requires a large amount of solvents.
Sterol concentrate purification, such as the evaporative fractionation disclosed in the European patent application EP 1 389 622 A2 does avoid salts and organic impurities, but again includes many process steps. The European patent EP 0 260 243 B1 describes a method to set free sterol from organic materials by treating said material with ammonia or amines in order to avoid the addition of inorganic salts which complicates further purification steps.

It has been an object of the present invention to provide an efficient economical, environmentally friendly process which provides a high process yield in the recovery of sterols and a high final sterol purity.

### Detailed description of the invention

The present invention provides a process for the recovery of sterols from organic material, which comprises
a) reacting organic material containing free sterols and sterol derivatives with
   (i) polyols or
   (ii) polyamines or
   (iii) alkanolamines or
   (iv) alcohols
   to convert the sterol derivatives into free sterols
b) reacting the residual reactants and their esters or amides with epoxydated sub-stances chosen from the group consisting of epoxydated soy bean oil, epoxydated linseed oil, epoxydated sunfloor oil, epoxydated lard oil, epoxydated fatty acids and epoxydated fatty acid esters such as tall oil fatty acids, oleic and linoleic acids; and subsequently
c) separating the free sterols from the remaining components by distillation.

As used herein the term "sterols" refers to any plant or animal based sterol or sterol derivative for example cholesterol, sitosterol, campesterol, ergosterol, stigmasterol, brassicasterol, ave-nasterol as well as the saturated components called stanols like sitostanol, stigmastanol and campestanol and their derivatives.

In nature sterols can occur as free sterols or predominantly derivatized as sterol esters of longchain fatty acids. During processing which is usually conducted at higher temperatures, low pressure, and relatively long residence time, most of the sterols in organic material are further derivatized.
Since sterol derivatives are difficult to separate or even purify either via distillation, extraction or direct crystallization it is necessary to convert them back to free sterols before proceeding with further processing steps. This high number of process steps renders a process uneconomical, so that one object of the current invention is the reduction of process steps. The enrichment of free sterols from natural organic material is achieved by step a) of the proposed process. Bonded sterols are converted back into free sterols using an appropriate reactant which combines a high transesterification or transamidation reaction yield with the conversion of fatty and other organic acids into their respective mono-, di- and polyesters, aminoamides, diamides, and polyaminoamides. Some reactants do neither require a catalyst, reactant excess nor high pressure to achieve complete transesterification or transamidation, in addition no solvent is used.

In order to prevent that the low molecular weight products formed or reactants left after transesterification or transamidation such as long chain alcohols and polyalcohols, glycols and polyglycols, amines and polyamines, alkanolamines, monoesters of polyalcohols, or ami-doamines will distill off together with the free sterols and contaminate the final product, the molecular weight of the non-free-sterol-material is increased. The crucial step of the process is therefore the reaction of the residual reactants and their respective alkanol esters or alkanolaminoamides left after step a) with epoxydated substances. The higher molecular weight of the residuants after reacting with the epoxyde groups results in a decreased vapor pressure, so that distillative separation of free sterols from the residuants is improved. This will avoid contamination with light end materials and increase the sterol concentration in the distillate. Free sterols can now easily be separated using a distillation apparatus with almost no theoretical separation stages such as molecular distillation, short path distillation or thin film evaporators. This process provides a distillate with a sufficient sterol concentration to produce sterols with a purity of more than 98% using only one single crystallization step.
Less reaction steps and a single distillation reduce processing costs significantly.

The organic material (feed) used in step a) can be any material containing free sterols or sterol esters or other sterol derivatives, such as vegetable oil distillates (VOD) and/or deodorizer distillates (DOD), residues of fatty acids (FAR) and/or fatty acid methyl or ethyl ester production (FAMER, FAEER), soap stock fatty acid residues (SSFAR), sugar cane wax (SCW), crude tall oil (CTO) and tall oil pitch (TOP). Due to its availability and amount of sterols or sterol derivatives the preferred feed is tall oil pitch.

Deodorizer distillate (DOD) or Vegetable Oil Distillate (VOD) from the deodorization process of vegetable oils, usually contain 2 - 15 wt % of sterols, using this type of feed concentrations of up to 45 wt % of free sterols based on the weight of the concentrate can be produced.

Residues from distillation of either fatty acids (FAR) or methyl esters (FAMER) contain 2 - 30 wt % of sterols and can also be concentrated up to 45 wt % free sterols based on the weight of the concentrate by the current technique, as well as crude tall oil (CTO) from the sulphatation employed in cellulose manufacturing, which contains 3 - 7 wt % sterol or tall oil pitch (TOP) derived from the process of paper production from wood which usually has 8 - 16 wt % sterols. Even other organic materials such as sugar cane waxes (SCW) and soap stock fatty acids (SSFA) with a sterol concentration below 5 wt % can be concentrated up to 45 wt % sterols based on the weight of the concentrate using the said process. Surprisingly it was found that the inventive process is always resulting in a high concentration of free sterols even if the organic material of step a) contains lower amounts of sterols. It is an important advantage that the yield of sterols from this process is independent from the organic material resp. the sterol concentration of the feed.

In step a) of the inventive process this organic material is reacted with long chain alcohols or polyalcohols, glycols or polyglycols, amines or polyamines, or alkanolamines and sterol esters and other sterol derivatives are transformed into the free sterols while other reactants result in the correspondent derivatives such as alkanol esters or amides.
Examples for polyols that could be used are
(i) polyols such as ethylene, diethylene glycol , triethylene, tetraethylene, and polyethylene glycols with molecular weights up to 1000, propylene glycol and polypropylene glycol having molecular weights up to 1000, glycerine and polyglycerines, mono and di - pentaerythritol, trimethylolpropane, high molecular weight polyols such as C7 to C24 fatty alcohols.
   Suitable polyamines are
(ii) ethylenediamine, triethylenetetramine, tetraethyleneamine or dimethylpropilamines.
   Alkanolamines for step a) could be
(iii) monoethanolamine , diethanolamine, triethalonamine, aminoethylethanolamine, dimethylaminopropylamine.
   The alcohols for this process step are
(iv) either from an Oxo process or linear alcohols from natural sources including the Guebert alcohols with C6 to C36 carbon atoms.
   The preferred reactants are polyamines, most preferred is ethylendiamine. The use of ethylendiamine enables the reaction without the use of a catalyst, avoiding solvents and high pressure.
   The amount of the reactants has to be adapted concerning the saponification value of the used feed. The molar ratio of reactant ((i) to (iv) ) in the transesterification and/or transamidation step (a) in relation to the saponification number of the organic material (feed) is 0,5 to 6.

The transesterification or transamidation is run in the presence of metallic catalysts, preferred are zinc oxide, sodium-, potassium- or lithium hydroxide and organic tin catalysts. Some reactants - such as alkanolamines, especially diethanolamine, do not require the use of a catalyst. Step a) and b) can be conducted in form of a batch process (examples 1 - 5 ) or in form of a continuous reaction process (examples 6, 7)
The batch process is conducted at temperatures of 150 to 290°C, preferably 180 to 270 °C, the best yield and purity is achieved at temperatures of 210 to 260 °C.
The continuous reaction process starts at ambient temperature of 20 to 30 °C and the temperature is increased up to 240 to 330 °C, preferably 250 to 310 °C and most preferred up to 270 to 300°C, while the pressure increases up to 1 to 25 bar, preferably 3 to 10 bar.

Step a) is followed by a quick reaction of the residual reactants and their respective alkanol esters or alkanolaminoamides left in the reaction product with the epoxy groups of the epoxydated substances.
The epoxydated substances are chosen from the group consisting of epoxy-dated vegetable oils such as epoxydated soy bean oil (ESO), epoxydated linseed oil (ELO), epoxydated sunflower oil or epoxydated lard oil or epoxydated fatty acids and epoxydated fatty acid esters such as tall oil fatty acids, oleic and linoleic acids. Most preferred are epoxydated soy bean oil (ESO) and/or epoxydated linseed oil (ELO).
In order to achieve the best results an amount of 2 to 20 wt % epoxydated vegetable oils preferably 5 to 10 wt % based on the amount of the feed used in step a) is added.

The temperature for the reaction of step b) is 120 to 220°C, preferably 150° to 200°C, especially 170°C to 190°C if the process is conducted as a batch process. In case of a continuous process the high temperature during step a) is rapidly cooled down after addition of the epoxydated material.

Without addition of the epoxydated substances the overall yield of free sterols is only 30 wt% while with step b) a free sterol yield of 40 to 45 wt % based on the weight of the concentrate can be achieved.

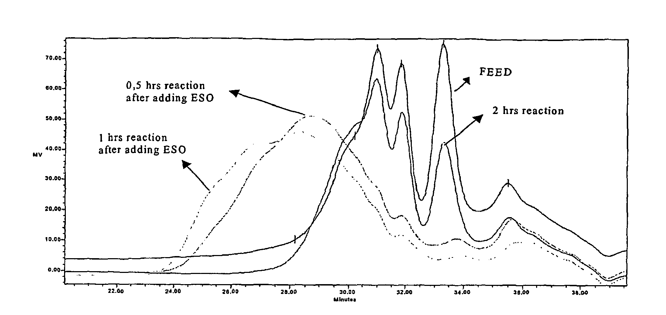

The result of a Gel Permeation Chromatography (GPC) in Figure 1 shows the process principle and the typical molecular weight growing profile from a typical feed during the transesterification or transamidation process, and after adding the epoxydated material.
(Line 1 represents the organic material (feed), line 2 the reaction product after 2 h reaction, line 3 is the reaction product 0,5 h after addition of ESO and line 4 represents the reaction product 1 h after addition of ESO)

Free sterols can then easily be separated using single distillation apparatus such as short path or thin film evaporation equipment, flash evaporators or molecular distillation. The possibility to use preferably short path distillation or molecular distillation enables the use of low distillation temperatures and low vacuum to avoid degradation and loss of sterols. The high reaction yield after step b) combined with a short path distillation results in a very short residence time avoiding sterols degradation or any other side reactions such as sterol ester formation and results in a process yield of more than 95 % based on the total amount of sterols.

Most preferred is a short path destillation, which is operated at a reduced pressure of 0.01-13.3 mbar (0.01 to 10 mm/Hg) and a temperature of 180 to 310 °C and/or a fractionation column which is operated at 0.13-6.6 mbar (0.1 to 5 mmHg) and a temperature of 170 to 230°C at the top and 240 to 280°C at the bottom.

After concentration of the sterols a final crystallisation process can be added as step d) in order to purify the sterols. Preferably this crystallisation does just include one crystallisation cycle. A sterol purification of 98 wt % and higher can be reached in just one single crystallisation step using the concentrated sterols resulting from step a to c).

### Examples

The examples describe the use of polyalcohols, long chain alcohols, polyamines and alkanolamines as reactants to set the sterols free by transesterification or transamidation of vegetable oil distillate (VOD), crude tall oil (CTO), tall oil pitch (TOP), fatty acid methyl ester residues (FAMER), sugar cane wax (SCW). After releasing the sterols and reaction of the residual reactants and their esters or amides with epoxydated products this material is distilled at high temperature and low pressure in order to separate the free sterols from the other high boiling point components.

### Step a) - Transesterification and/or Transamidation Step and step b)

### Example 1

1 kg of TOP were reacted with 155 g (2.53 moles) monoethanolamine from Aldrich Chemical Co. and 10 g zinc oxide (0.12 moles) from Merck KGaA, in a 2-liter, 3-necked round bottom flask equipped with a thermometer and mechanical agitator at a temperature of 220 °C for 5 h. Then 50 g of epoxydated soy bean oil (ESO) were added to the flask, reaction temperature was maintained at 180° C and the reaction was continued for 1 hour. The yield of sterol release - relation of free sterols in the reaction product after step a and b compared to the total amount of free and derivatized sterols in the feed - was 99,34%.

### Example 2

1 kg of VOD was treated with 326 g (3,1 moles) of diethyleneglycol from Aldrich Chemical Co. and 17 g of zinc oxide (0.21 moles) from Merck KGaA, in a 2-liter, 3-necked round bottom flask equipped with a thermometer and mechanical agitator at a temperature of 220 °C. After 5 hours 80 g of ESO were added to the flask, reaction temperature was maintained at 180° C and the reaction was continued for 1 hour. The yield of sterol release was 90,74%.

### Example 3

1 kg of TOP was reacted with 60 g (1,00 moles) of ethylenediamine from Aldrich Chemical Co. into a 2-liter, 3-necked round bottom flask equipped with a thermometer and mechanical agitator at a temperature of 220 °C for 3 h. After reaction completion 70 g of ESO were added to the flask, reaction temperature was maintained at 180° C and the reaction was continued for 1 hour. The yield of sterol liberation was 99,7%.

### Example 4

1 kg of FAMER was reacted with 470 g (5,11 moles) of Glycerol from Aldrich Chemical Co. and 1,7 g of Lithium Hydroxide (1,0 mol) from Merck KGaA, in a 2-liter, 3-necked round bottom flask equipped with a thermometer and mechanical agitator at a temperature of 240 °C. After 5 h 60g of ESO were added to the flask, reaction temperature was maintained at 180° C and the reaction was continued for 1 hour. The yield of sterol liberation was 85%.

### Example 5

1 kg of VOD was reacted with 90 g of ethylenediamine ( 1,5 moles) obtained from Aldrich Chemical Co., in a 2-liter, 3-necked round bottom flask equipped with a thermometer and mechanical agitator at a temperature of 220 °C. After 4 hours 90 g of ESO were added to the flask, reaction temperature was maintained at 180° C and the reaction was continued for 1 hour. The yield of sterol release was 99,5%.

### Example 6

0,9 kg of TOP were mixed with 60 g of ethylenediamine ( 1,0 moles) obtained from Aldrich Chemical Co., and transferred into a pressure reactor. Within 20 minutes temperature was increased from initially 25°C to 290° C and maintained for another 20 minutes, a maximum pressure of 6 bar was achieved. The pressure was released to atmospheric pressure and 60 g of ESO were added, the temperature was kept for another 5 minutes and then cooled down to ambient temperature (25°C) within 25 minutes. The yield of sterol release was 98,0%.

### Example 7

0,9 kg of TOP were mixed together with 60 g of ethylenediamine ( 1,0 moles) obtained from Aldrich Chemical Co., and transferred into a pressure reactor. Within 20 minutes temperature was increased from initially 25°C to 275° C and maintained for another 20 minutes, a maximum pressure of 4 bar was achieved. The pressure was released to atmospheric pressure and 60 g of ESO were added, the temperature was kept for another 5 minutes and then cooled down to ambient temperature (25°C) within 25 minutes. The yield of sterol release was 95,0%.

### Step c) - Concentrating the Sterols using Short Path Distillation:

### Example 8

1 kg of the product from example 1 was distilled off in a short path evaporator (SPE) which was operated at 0.13 mbar (0.1 mm Hg), 290 °C and a feed flow of 600ml/hour. The residue 1 leaving the bottom of the SPE represented 55% w/w of the CTO feed. The top fraction 2 was representing 45% w/w with 24 % of free sterols. The sterols recover yield in this step - amount of concentrated free sterols after distillation compared to amount of free sterols from steps a and b used for distillation - was 83 %.

### Example 9

1 kg of the product from example 3 was distilled off in a SPE which was operated at 0.13 mbar (0,1 mm Hg) 290°C and a feed flow of 600ml/hour. The residue 1 leaving the bottom of the WFE represented 67% w/w of the feed. The top fraction 2 was representing 33% w/w with 40% of free sterols. The sterols recover yield in this step was 97,1%.
This sterols have further been purified by a one step crystallization:
80 g of the crude sterols were dissolved in 184 g of n-heptane at 65°C in a jacketed glass reactor of 1000 ml with a diameter of 105 mm equipped with a KPG-stirrer (plate stirrer) and a reflux cooler. After achieving a clear solution 16 g of methanol / DI-water (50/50wt%) was added. Crystallization of sterols started immediately. The slurry was further cooled down to a product temperature of 25°C in 40 minutes. The crystallized slurry was filtered using the Buchner vacuum funnel to filtrate off the mother liquor. The residual cake was washed two times with 100 ml of fresh n-heptane at ambient temperature. The final sterol potency was 98.2%.

### Example 10

1 kg of the product from example 2 was distilled off in a SPE which was operated at 0.13 mbar (0,1 mm Hg), 290 °C and a feed flow of 600ml/hour. The residue 1 leaving the bottom of the WFE represented 73,4% w/w of the feed. The top fraction 2 was representing 26,6 % w/w with 28,6 % of free sterols. The sterols recover yield in this step was 73%.

### Example 11

0,9 kg of the product from example 6 was distilled off in a SPE which was operated at 0.13 mbar (0,1 mm Hg), 280 °C and a feed flow of 600ml/hour. The residue 1 leaving the bottom of the WFE represented 67% w/w of the feed. The top fraction 2 was representing 33% w/w with 38% of free sterols. The sterols recover yield in this step was 94,8%.
These sterols have further been purified by a one step crystallization:
80 g of the crude sterols were dissolved in 192 g of n-hexane at 65°C in a jacketed glass reactor of 1000 ml with a diameter of 105 mm equipped with a KPG-stirrer (plate stirrer) and a reflux cooler. After achieving a clear solution 8 g of methanol / DI-water (50/50wt%) was added. Crystallization of sterols started immediately. The slurry was further cooled down to a product temperature of 25°C in 40 minutes. The crystallized slurry was filtered using the Buchner vacuum funnel to filtrate off the mother liquor. The residual cake was washed two times with 100 ml of fresh n-hexane at ambient temperature. The final sterol purity was 98.5%.

## Claims

1. Process for the recovery of sterols from organic material, which comprises
a) reacting organic material containing free sterols and sterol derivatives with
(i) polyols or
(ii) polyamines or
(iii) alkanolamines or
(iv) alcohols
to convert the sterol derivatives into free sterols
b) reacting the residual reactants and their esters or amides with epoxydated substances chosen from the group consisting of epoxydated soy bean oil, epoxydated linseed oil, epoxydated sunfloor oil, epoxydated lard oil, epoxydated fatty acids and epoxydated fatty acid esters such as tall oil fatty acids, oleic and linoleic acids; and subsequently
c) separating the free sterols from the remaining components by distillation.

2. Process according to claim 1, wherein the sterol containing organic material is chosen from the group consisting of vegetable oil distillates and/or deodorizer distillates, residues of fatty acids and/or fatty acid methyl ester production, soap stock fatty acid residues, sugar cane wax, crude tall oil and tall oil pitch.

3. Process according to claims 1 and/or 2, wherein the epoxydated substances are added in an amount of 2 to 20 wt % based on the amount of organic material (feed) used in step a).

4. Process according to claims 1 to 3, wherein step a) is carried out in the presence of a catalyst selected from the group consisting of metal catalysts such as organo tin catalysts, zinc oxide and hydroxides of sodium, potassium or lithium.

5. Process according to claims 1 to 4, wherein step a) is carried out without the addition of a catalyst.

6. Process according to claims 1 to 5, **characterised in that** step a) and b) is conducted in form of a batch process or as continuous process.

7. Process according to claims 1 to 6, wherein the transesterification and/or transamidation of step a) is conducted at a temperature of 150 to 290 °C (batch process).

8. Process according to claims 1 to 6, wherein the transesterification and/or transamidation of step a) is conducted at increasing temperatures starting at 20 to 30 °C and rising up to 240 °C to 330 °C (continuous process).

9. Process according to claims 1 to 8, wherein step b) is conducted at a temperature of 120 to 220 °C.

10. Process according to claims 1 to 9, wherein the molar ratio of reactant ((i) to (iv) ) in the transesterification and/or transamidation step (a) in relation to the saponification number of the organic material (feed) is 0,5 to 6.

11. Process according to claims 1 to 10, wherein step c) is conducted by short path distillation or thin film evaporators or flash evaporators or molecular distillation.

12. Process according to claims 1 to 11, **characterised in that** a crystallisation is carried out after step c) for final purification of the sterols.

## Patentansprüche

1. Verfahren zur Gewinnung von Sterolen aus organischem Material, bei dem man
a) freie Sterole und Sterolderivate enthaltendes organisches Material mit
(i) Polyolen oder
(ii) Polyaminen oder
(iii) Alkanolaminen oder
(iv) Alkoholen
umsetzt, wodurch die Sterolderivate in freie Sterole umgewandelt werden,
b) die verbliebenen Reaktanten und ihre Ester oder Amide mit epoxidierten Substanzen aus der Gruppe bestehend aus epoxidiertem Sojabohnenöl, epoxidiertem Leinöl, epoxidiertem Sonnenblumenöl, epoxidiertem Lardöl, epoxidierten Fettsäuren und epoxidierten Fettsäureestern wie Tallölfettsäuren, Ölsäure und Linolsäure umsetzt und danach
c) die freien Sterole durch Destillation von den übrigen Komponenten abtrennt.

2. Verfahren nach Anspruch 1, bei dem man das Sterol enthaltende organische Material aus der Gruppe bestehend aus Pflanzenöldestillaten und/oder Desodorisatordestillaten, Rückständen von Fettsäuren und/oder der Fettsäuremethylesterproduktion, Seifenstockfettsäurerückständen, Zuckerrohrwachs, rohem Tallöl und Tallpech auswählt.

3. Verfahren nach Anspruch 1 und/oder 2, bei dem man die epoxidierten Substanzen in einer Menge von 2 bis 20 Gew.-%, bezogen auf die in Schitt a) verwendete Menge an organischem Material (Feed), zugibt.

4. Verfahren nach einem der Ansprüche 1 bis 3, bei dem man Schritt a) in Gegenwart eines Katalysators aus der Gruppe bestehend aus Metallkatalysatoren wie Organozinnkatalysatoren, Zinkoxid und Hydroxiden von Natrium, Kalium oder Lithium durchführt.

5. Verfahren nach einem der Ansprüche 1 bis 4, bei dem man Schritt a) ohne Zugabe eines Katalysators durchführt.

6. Verfahren nach einem der Ansprüche 1 bis 5, **dadurch gekennzeichnet, daß** man Schritt a) und Schritt b) in Form eines diskontinuierlichen Verfahrens oder als kontinuierliches Verfahren durchführt.

7. Verfahren nach einem der Ansprüche 1 bis 6, bei dem man die Umesterung und/oder Umamidierung von Schritt a) bei einer Temperatur von 150 bis 290°C durchführt (diskontinuierliches Verfahren).

8. Verfahren nach einem der Ansprüche 1 bis 6, bei dem man die Umesterung und/oder Umamidierung von Schritt a) bei zunehmenden Temperaturen, die bei 20 bis 30°C beginnen und auf 240°C bis 330°C ansteigen, durchführt (kontinuierliches Verfahren).

9. Verfahren nach einem der Ansprüche 1 bis 8, bei dem man Schritt b) bei einer Temperatur von 120 bis 220°C durchführt.

10. Verfahren nach einem der Ansprüche 1 bis 9, bei dem das Molverhältnis von Reaktant ((i) bis (iv)) im Umesterungs- und/oder Umamidierungsschritt (a) in Bezug auf die Verseifungszahl des organischen Materials (Feed) 0,5 bis 6 beträgt.

11. Verfahren nach einem der Ansprüche 1 bis 10, bei dem man Schritt c) durch Kurzwegdestillation, Dünnschichtverdampfer oder Flashverdampfer oder Molekulardestillation durchführt.

12. Verfahren nach einem der Ansprüche 1 bis 11, **dadurch gekennzeichnet, daß** man nach Schritt c) eine Kristallisation zur abschließenden Reinigung der Sterole durchführt.

## Revendications

1. Procédé pour la récupération de stérols à partir de matière organique, qui consiste
a) à faire réagir une matière organique contenant des stérols libres et des dérivés de stérols avec :
(i) des polyols ou
(ii) des polyamines ou
(iii) des alcanolamines ou
(iv) des alcools
pour convertir les dérivés de stérols en stérols libres,
b) à faire réagir les réactifs résiduels et leurs esters ou amides avec des substances époxydées choisies dans le groupe constitué de l'huile de soja époxydée, l'huile de lin époxydée, l'huile de tournesol époxydée, l'huile de saindoux époxydée, des acides gras époxydés et des esters d'acides gras époxydés tels que les acides gras d'huile de tall , les acides oléique et linoléique, et ensuite
c) à séparer les stérols libres des composants restants par distillation.

2. Procédé selon la revendication 1,
selon lequel
la matière organique contenant des stérols est choisie dans le groupe constitué de distillats d'huiles végétales et/ou distillats de désodorisants, résidus de production d'acides gras et/ou d'esters méthyliques d'acides gras, résidus d'acides gras de savon, cire de canne à sucre, huile de tall brute et poix d'huile de tall .

3. Procédé selon les revendications 1 et/ou 2, selon lequel
les substances époxydées sont ajoutées dans une proportion de 2 % à 20 % en poids sur la base de la quantité de matière organique (alimentation) utilisée dans l'étape a).

4. Procédé selon les revendications 1 à 3,
selon lequel
l'étape a) est mise en oeuvre en présence d'un catalyseur choisi dans le groupe constitué de catalyseurs métalliques tels que des catalyseurs organo-étain, l'oxyde de zinc, et les hydroxydes de sodium, potassium ou lithium.

5. Procédé selon les revendications 1 à 4
selon lequel
l'étape a) est mise en oeuvre sans l'addition d'un catalyseur.

6. Procédé selon les revendications 1 à 5,
**caractérisé en ce que**
l'étape a) et l'étape b) sont mises en oeuvre sous forme d'un procédé discontinu ou d'un procédé continu.

7. Procédé selon les revendications 1 à 6,
selon lequel
la transestérification et/ou la transamidation de l'étape a) est/sont effectuée(s) à une température de 150 °C à 290 °C (procédé discontinu).

8. Procédé selon les revendications 1 à 6,
selon lequel
la transestérification et/ou la transamidation de l'étape a) est (sont) effectuée(s) à température croissante commençant à 20 °C jusqu'à 30 °C et s'élevant jusqu'à 240 °C et à 330 °C. (procédé continu).

9. Procédé selon les revendications 1 à 8,
selon lequel l'étape
b) est mise en oeuvre à une température de 120 à 220° C.

10. Procédé selon les revendications 1 à 9,
selon lequel
le rapport molaire des réactifs ((i) à (iv)) dans l'étape de transestérification et/ou de transamidation (a) en relation avec l'indice de saponification de la matière organique (alimentation) est de 0,5 à 6.

11. Procédé selon les revendications 1 à 10,
selon lequel l'étape
c) est mise en oeuvre par distillation courte ou avec des évaporateurs à film mince ou des évaporateurs flash ou par distillation moléculaire.

12. Procédé selon les revendications 1 à 11,
**caractérisé en ce qu'**
une cristallisation est effectuée après l'étape c) pour la purification finale des stérols.
